(19) Europäisches Patentamt
European Patent Office
Office européen des brevets

(11) Publication number: 0 142 811
A2

# (12) EUROPEAN PATENT APPLICATION

(21) Application number: 84113655.9

(22) Date of filing: 12.11.84

(51) Int. Cl.⁴: C 07 D 487/04
//(C07D487/04, 249:00, 239:00)

(30) Priority: 14.11.83 US 551540

(43) Date of publication of application:
29.05.85 Bulletin 85/22

(84) Designated Contracting States:
AT BE CH DE FR GB IT LI NL SE

(71) Applicant: THE DOW CHEMICAL COMPANY
Dow Center 2030 Abbott Road Post Office Box 1967
Midland Michigan 48640(US)

(72) Inventor: Mckendry, Lennon H.
5314 Campau Drive
Midland Michigan 48640(US)

(72) Inventor: Pearson, Norman R.
141-3 Roxanne Court
Walnut Creek California 94596(US)

(74) Representative: Weickmann, Heinrich, Dipl.-Ing. et al,
Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820
D-8000 München 86(DE)

## (54) Method of preparing 1,2,4-triazolo 1,5-a - pyrimidine-2-sulfonyl chlorides.

(57) 1,2,4-Triazolo[1,5-a] pyrimidine-2-sulfonyl chloride compounds are prepared by reacting a 1,2,4-triazolo[1,5-a] pyrimidine-2-mercapto (or arylmethylthio) compound with an effective amount of a hypochlorite solution in a two phase solvent system comprising a aqueous acidic phase and an organic phase under conditions sufficient to cause formation of the desired sulfonyl chloride compounds.

EP 0 142 811 A2

0142811

# METHOD OF PREPARING 1,2,4-TRIAZOLO[1,5-a]-PYRIMIDINE-2-SULFONYL CHLORIDES

The present invention relates to a method of preparing 1,2,4-triazolo[1,5-a]-pyrimidine-2--sulfonyl chlorides by reacting a 1,2,4-triazolo-[1,5-a]pyrimidine-2-mercapto (or arylmethylthio) compound with a hypochlorite solution in a two phase solvent system.

Sulfonyl chlorides have generally been prepared employing $Cl_2$ and acetic acid-water. $Cl_2$ is a particularly troublesome reagent to work with because it is a strong irritant both by contact and by inhalation. Furthermore, the use of $Cl_2$ is incompatible with certain functional groups, such as methoxy, that may be present on the pyrimidine portion of the triazolo-pyrimidine starting material. The present invention remedies the above-identified problems associated with the preparation of sulfonyl chlorides employing $Cl_2$ and further provides improved and more reproducible yields of 1,2,4-triazolo[1,5-a]pyrimidine--2-sulfonyl chlorides when compared to prior art methods of preparation.

In accordance with the present invention, 1,2,4-triazolo[1,5-a]pyrimidine-2-sulfonyl chlorides are prepared by reacting a 1,2,4-triazolo[1,5-a]-pyrimidine-2-mercapto (or arylmethylthio) compound with an effective amount of a hypochlorite solution in a two phase solvent system comprising an aqueous acidic phase and a water immiscible organic phase under conditions sufficient to cause formation of the desired 1,2,4-triazolo[1,5-a]pyrimidine-2-sulfonyl chloride compound in a good yield. The desired product is then isolated from the organic phase employing standard, well-known, separatory and purification techniques. The 1,2,4-triazolo[1,5-a]pyrimidine-2-sulfonyl chlorides are useful as chemical intermediates in the preparation of sulfonamide herbicides.

In the practice of the present invention, it is essential to employ: (1) a 1,2,4-triazolo[1,5-a]-pyrimidine-2-mercapto (or arylmethylthio) compound (2) an effective amount of a hypochlorite solution and (3) a 2-phase solvent system comprising an aqueous acidic phase and a water-immiscible organic phase.

Suitable 1,2,4-triazolo[1,5-a]pyrimidine compounds useful as starting materials include compounds of the formula

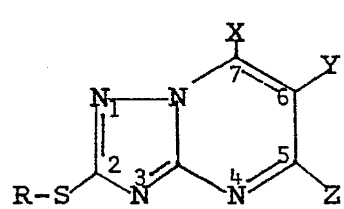

(I)

wherein

R represents H or an arylmethyl (Aryl $CH_2$-) group such as benzyl;

X, Y and Z independently represent H, alkyl, haloalkyl, alkoxy, haloalkoxy or halo with the proviso that X and Y or Y and Z can be joined together in a cyclic structure. Advantageously, X, Y and Z independently represent H, $C_1$-$C_4$ alkyl, $C_1$-$C_4$ alkoxy and X and Y or Y and Z can be joined to form a cycloalkyl ring or a ring containing a heteroatom. Preferred compounds include those compounds wherein Y is H and X and Z independently represent hydrogen, $C_1$-$C_4$ alkyl or $C_1$-$C_4$ alkoxy and those compounds wherein Z represents $C_1$-$C_4$ alkyl and X and Y are joined to form a cyclopentano group.

Hypochlorite solution, when used herein, is meant to encompass an aqueous solution of a metallic salt of hypochlorous acid. Suitable hypochlorite solutions, for use in the present invention, include NaOCl, KOCl, LiOCl and $Ca(OCl)_2$ with NaOCl being preferred. The concentration of the hypochlorite solution is usually in the range of from 3 to 15 weight percent. For convenience and economy, commercial bleach is advantageously employed as the source of NaOCl. An effective amount of hypochlorite solution is achieved by providing at least 3 molar equivalents of metal hypochlorite per molar equivalent of triazolo-pyrimidine starting material present in the reaction. Advantageously an excess of hypochlorite is employed in the range of from 3 to 10 molar equivalents per mole of triazolo-pyrimidine compound and preferably between 3 and 4 molar equivalents per mole of triazolo-pyrimidine compound.

The 2-phase solvent system comprises an aqueous acidic phase and a water-immiscible organic phase. The ratio of organic phase to aqueous phase is not critical but will usually be in the range of from 0.5:1 to 20:1 by volume (organic:aqueous). Advantageously the aqueous acidic phase is an aqueous mineral acid solution such as hydrochloric acid (HCl), sulfuric acid ($H_2SO_4$) or phosphoric acid ($H_3PO_4$). Preferred mineral acids include HCl and $H_2SO_4$. When a mineral acid is employed as the aqueous acidic phase it is preferred to employ a molar quantity of mineral acid about equivalent to the molar amount of metal hypochlorite present in the reaction.

The water-immiscible organic phase can be any water-immiscible organic solvent in which the triazolo sulfonyl chloride product is soluble. Suitable water-immiscible organic solvents include aliphatic or aromatic hydrocarbons and chlorinated aliphatic or aromatic hydrocarbons. Preferred organic solvents include methylene chloride ($CH_2Cl_2$), carbon tetrachloride ($CCl_4$), chloroform ($CHCl_3$), chlorobenzene or 1,1,1-trichloroethane ($CH_3CCl_3$). The organic solvent must be present in the present reaction in an amount sufficient to dissolve all of the sulfonyl chloride reaction product. Usually, the organic solvent is present in from 2 to 100 parts by weight of the triazolo-pyrimidine starting material, advantageously from 5 to 50 parts by weight triazolo-pyrimidine starting material and preferably from 10 to 25 parts by weight triazolo-pyrimidine starting material.

The present invention can be conducted at room temperature but temperatures below room temperature

generally result in higher yields. Advantageously, the present reaction is conducted at a temperature of from -10°C to 10°C and preferably from -5°C to 0°C. The present reaction is typically conducted in the presence of agitation sufficient to cause a thorough contacting of the reactants. It is not necessary that the agitation cause a uniform dispersion of the aqueous acidic phase and the organic phase although severe agitation accomplishing such a dispersion may be employed.

In conducting the present reaction, neither the rate of addition nor the order of addition of the reactants is critical. Preferably, the 1,2,4-triazolo-[1,5-a]pyrimidine-2-mercapto (or arylmethylthio) starting material, aqueous acidic phase and organic phase are combined, cooled to the desired temperature and the hypochlorite solution is thereafter added slowly, with agitation, so that the temperature remains in the desired range. A typical reaction is usually complete in from 0.25 to 4 hours.

The present reaction can be characterized by the following chemical equation:

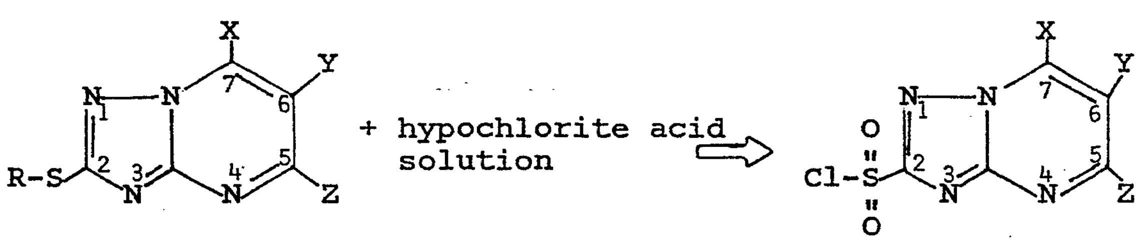


wherein R, X, Y and Z are defined hereinabove.

After completion of the present reaction the desired sulfonyl chloride product is isolated from the organic phase employing standard separatory

and purification techniques such as, for example, distillation of the solvent, trituration and recrystallization. When R represents arylmethyl, the aryl by-products are preferably removed from the concentrated reaction mixture or crude product by trituration of the crude product with a suitable hydrocarbon solvent, such as, for example, hexane, pentane, cyclohexane or methylcyclohexane. After trituration, the reaction mixture is filtered resulting in the desired solid sulfonyl chloride product.

In one embodiment of the present invention, 5,7-dimethyl-2-mercapto-1,2,4-triazolo[1,5-a]pyrimidine is added to a 2-phase solvent system of water and methylene chloride. The mixture is cooled to -5°C to 0°C and concentrated HCl (37.5% in water) is added. After addition of the HCl, an effective amount of sodium hypochlorite (NaOCl) solution is slowly added to the reaction mixture with stirring while maintaining the temperature between -5°C and 0°C. After the addition of the NaOCl, the reaction mixture is agitated, until the reaction is complete, i.e., usually from 1/4 hour to 4 hours. The phases are then separated and the aqueous phase is extracted with $CH_2Cl_2$. The organic phases ($CH_2Cl_2$) are combined, washed with aqueous sodium bisulfite and dried with $Na_2SO_4$ and $MgSO_4$. Evaporation of the solvent results in formation of the product as a white solid.

In another embodiment, 5,7-dimethoxy-2-benzylthio-1,2,4-triazolo[1,5-a]pyrimidine is added to a 2-phase solvent system of water and $CH_2Cl_2$. The mixture is cooled to -5°C to 0°C and concentrated HCl (37.5% in water) is added. After addition of the HCl,

an effective amount of sodium hypochlorite solution (NaOCl) is slowly added to the reaction mixture with stirring while maintaining the temperature between -5°C and 0°C. After the addition of the NaOCl, the reaction mixture is agitated until the reaction is complete, i.e., usually from 1/4 hour to 4 hours. The phases are then separated and the aqueous phase is extracted with $CH_2Cl_2$. The organic phases are combined, washed with aqueous sodium bisulfite and dried over $MgSO_4$. Evaporation of the solvent results in the formation of a yellow oil. Upon trituration of the yellow oil with hexane, the desired product is recovered as a gummy yellow solid.

The following examples further illustrate the present invention. No attempt has been made to balance any chemical equation described herein.

Example 1: Preparation of 5,7-dimethyl-1,2,4-triazolo-[1,5-a]pyrimidine-2-sulfonyl chloride

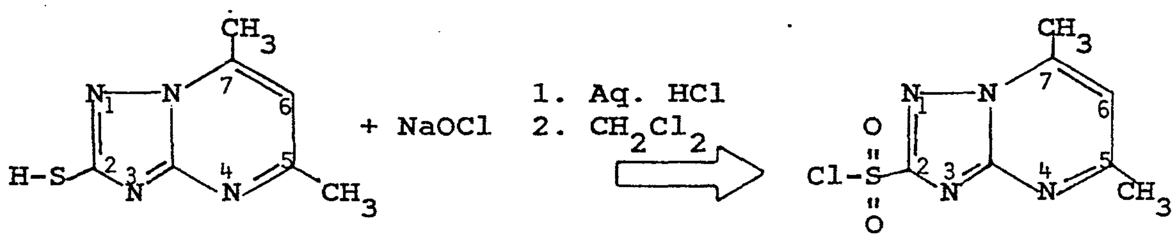


To 80.1 mg (0.444 mmol) of 5,7-dimethyl-2-mercapto-1,2,4-triazolo[1,5-a]pyrimidine in 2.0 ml of methylene chloride ($CH_2Cl_2$) and 1.0 ml of water, was added, after cooling to 5°C, 0.30 ml (3.6 mmol) of concentrated HCl (37.5% in water). The mixture was stirred well and 5.0 ml (3.5 mmol) of aqueous sodium hypochlorite (5.25%) was added dropwise over a 15

minute period while maintaining the temperature at less than 5°C. The mixture was stirred for another 15 minutes at 5°C and then the phases were separated. The aqueous phase was extracted with $CH_2Cl_2$ and the combined $CH_2Cl_2$ layers were washed with aqueous sodium bisulfite and dried with $Na_2SO_4$ and $MgSO_4$. Evaporation of the solvent afforded 102 mg (94%) of a white solid which was characterized by nuclear magentic resonance (NMR) spectroscopy in comparison to an authentic sample.

<u>Example 2</u>: Preparation of 5,7-dimethyl-1,2,4-triazolo-[1,5-a]pyrimidine-2-sulfonyl chloride

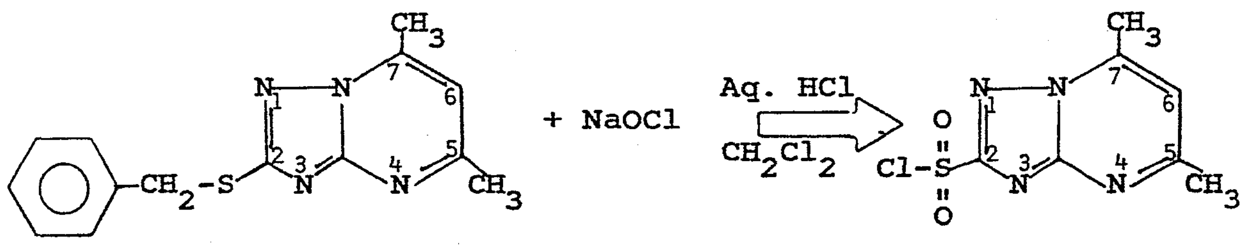


A mixture of 2.00 g (7.40 mmol) of 5,7-dimethyl--2-benzylthio-1,2,4-triazolo[1,5-a]pyrimidine, 33 ml of methylene chloride ($CH_2Cl_2$), 17 ml of water and 2.1 ml (25 mmol) of concentrated HCl (37.5% in water) was stirred and cooled to -5°C. To this mixture was added dropwise 35.0 ml (24.5 mmol) of aqueous sodium hypochlorite (5.25%) over a 15 minute period while maintaining the temperature at less than 0°C. The yellow mixture was stirred an additional 10 minutes at -5°C to 0°C and then the phases were separated. The aqueous phase was extracted with methylene chloride and the combined organic layers were washed with aqueous sodium bisulfite and dried over magnesium sulfate. Evaporation of the solvent afforded an oil that began to solidify. Trituration with hexane and filtration gave 1.56 g

(85%) of a light brown solid which was characterized by NMR spectroscopy. A sample of the sulfonyl chloride product was purified by dissolving it in hot acetone, filtering, rotoevaporating to dryness and recrystallizing from ethyl acetate to provide an off-white solid with a melting point (m.p.) of 128.5-130.5°C. Analysis of the product was:

| | carbon | hydrogen | nitrogen | sulfur |
|---|---|---|---|---|
| Calculated: | 34.09 | 2.86 | 22.71 | 13.00 |
| Found: | 34.34 | 2.80 | 22.64 | 12.85 |

Example 3 Preparation of 5,7-dimethoxy-1,2,4-triazolo-[1,5-a]pyrimidine-2-sulfonyl chloride

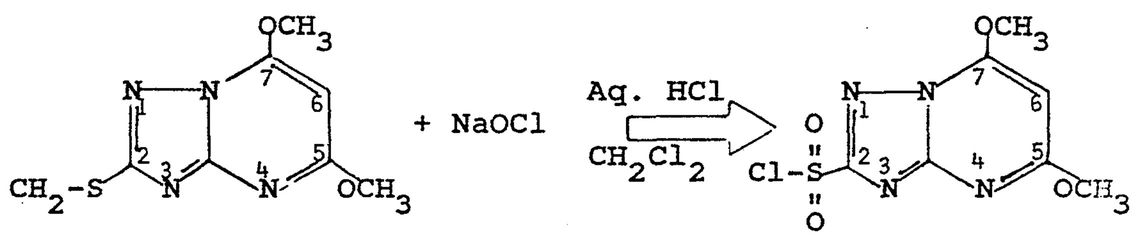


A mixture of 2.00 g (6.62 mmol) of 5,7-dimethoxy-2-benzylthio-1,2,4-triazolo[1,5-a]pyrimidine, 30 ml of methylene chloride ($CH_2Cl_2$) and 15 ml of water was stirred and cooled to -5°C. 1.90 ml (22.5 mmol) of concentrated HCl (37.5% in water) was added to the reaction mixture. Aqueous sodium hypochlorite (5.25%; 31.0 ml; 21.9 mmol) was added dropwise to the reaction mixture over a 15 minute period while maintaining the temperature at -5° to 0°C. After stirring an additional 20 minutes at 0°C, the yellow mixture was separated and the aqueous phase extracted with methylene chloride. The combined organic layers were then washed with aqueous sodium bisulfite and

dried over magnesium sulfate. Evaporation of the solvent furnished a yellow oil which upon trituration with hexane gave 1.8 g (97% crude) of a gummy, yellow solid. The crude sulfonyl chloride product was characterized by NMR.

Example 4 Preparation of 5-methyl-1,2,4-triazolo[1,5-a]-pyrimidine-2-sulfonyl chloride

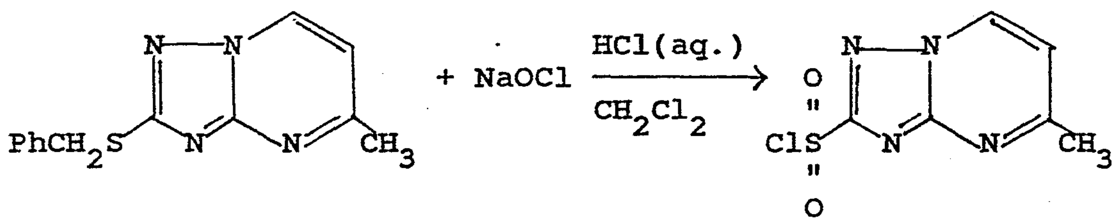


A two phase system of 9.0 g (35 mmol) of 2-benzylthio-5-methyl-1,2,4-triazolo[1,5-a]pyrimidine and 10.8 ml (130 mmol) of conc. HCl in 180 ml of $CH_2Cl_2$ and 90 ml of $H_2O$ was cooled to -5°C. Aqueous sodium hypochlorite (5.25%, 180 ml, 128 mmol) was added dropwise and the resultant solution was stirred for 15 minutes at -5° to 0°C. The stirring was stopped and the organic phase was separated. The aqueous phase was washed twice with $CH_2Cl_2$, and the combined organic phases were washed with 10% $NaHSO_3$ (aq.), dried ($MgSO_4$) and concentrated in vacuo to yield a yellow oil. The oil was triturated with pentane to separate a solid. The solid was collected by filtration and dried in vacuo to yield 7.52 g (92%) of the desired product as an orange solid characterized by IR and 'H NMR.

1. A method of preparing a 1,2,4-triazolo-[1,5-a]pyrimidine-2-sulfonyl chloride compound which comprises reacting a 1,2,4-triazolo[1,5-a]pyrimidine-2-mercapto (or arylmethylthio) compound with an effective amount of a hypochlorite solution in a two phase solvent system comprising an aqueous acidic phase and a water-immiscible organic phase under conditions sufficient to cause formation of the desired 1,2,4-triazolo[1,5-a]-pyrimidine-2-sulfonyl chloride compound.

2. The method of Claim 1 wherein the aqueous acidic phase is an aqueous mineral acid and the water-immiscible organic phase is an aliphatic hydrocarbon or a chlorinated aliphatic hydrocarbon.

3. The method of Claim 2 wherein the reaction is conducted at a temperature of from -10°C to 10°C.

4. The method of Claim 3 wherein the 1,2,4-triazolo[1,5-a]pyrimidine-2-mercapto (or arylmethylthio) starting material is a compound of the formula

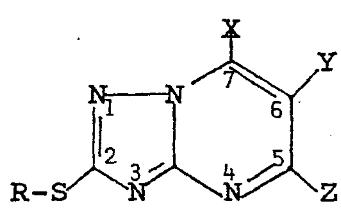

wherein

R represents hydrogen or arylmethyl;

X, Y and Z independently represent hydrogen, alkyl, alkoxy, haloalkyl, haloalkoxy or halo with the proviso that X and Y or Y and Z can be joined to form a cyclic structure.

5. The method of Claim 4 wherein X, Y and Z independently represent methyl or hydrogen.

6. The method of Claim 5 wherein X and Z represent methyl and Y represents hydrogen and the desired product corresponds to the formula

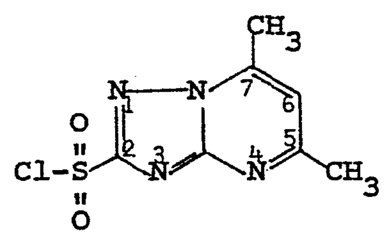

7. The method of Claim 5 wherein X and Y represent hydrogen and Z represents methyl and the desired product corresponds to the formula

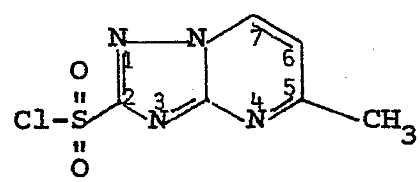

8. Method of any one of Claims 1 to 7 which comprises reacting 5,7-dimethyl-2-mercapto-1,2,4-triazolo[1,5-a]pyrimidine with an effective amount of a hypochlorite solution in a 2-phase solvent system

comprising an aqueous acidic phase and a water-immiscible organic phase under conditions sufficient to cause formation of 5,7-dimethyl-1,2,4-triazolo[1,5-a]-pyrimidine-2-sulfonyl chloride.

9. Method of any one of Claims 1 to 7 which comprises reacting 5-methyl-2-benzylthio-1,2,4-triazolo-[1,5-a]pyrimidine with an effective amount of a hypochlorite solution in a 2-phase solvent system comprising an aqueous acidic phase and a water-immiscible organic phase under conditions sufficient to cause formation of 5-dimethyl-1,2,4-triazolo[1,5-a]pyrimidine-2-sulfonyl chloride.